# EUROPEAN PATENT APPLICATION

(11) **EP 4 523 699 A1**
(43) Date of publication of application: **19.03.2025**
(21) Application number: 24199275.9
(22) Date of filing: 09.09.2024
(51) Int. Cl.: A61K 38/40, A61K 31/683, A61K 31/00, A61K 45/06, A61P 17/00

(54) **ANTI-INFLAMMATORY DERMATOLOGICAL COMPOSITION COMPRISING GLYCEROPHOSPHOINOSITOL, A LACTOFERRIN HYDROLYSATE AND A CANNABINOID, IN PARTICULAR FOR TREATING SEBORRHEIC DERMATITIS**

(30) Priority: 15.09.2023 IT 202300019032
(71) Applicant: FB Dermo srl, 20149 Milano (IT)
(72) Inventor: BANFI, Fabio, Milano (IT)
(74) Representative: Manfredi, Irene

(57) **Abstract**

The present invention relates to a composition comprising: a) glycerophosphoinositol (GPI), or a derivative thereof, or a salt thereof; b) a lactoferrin hydrolysate; and c) at least one cannabinoid, able to offer significant relief in the treatment of skin diseases, in particular of inflammatory skin diseases. Furthermore, the present invention also relates to pharmaceutical products comprising said composition of the invention with suitable pharmaceutically acceptable excipients, and to the use of the same in the treatment of skin diseases.

## Description

### FIELD OF THE INVENTION

The present invention relates to a composition able to offer significant relief in the treatment of skin diseases, in particular of inflammatory skin diseases. Furthermore, the present invention also relates to pharmaceutical products comprising said composition of the invention with suitable pharmaceutically acceptable excipients, and to the use of the same in the treatment of skin diseases.

### BACKGROUND ART

The skin acts as a barrier to protect the human body blocking intrusion factors from the outside. Physicochemical stimulation, allergens, ultraviolet rays, oxidative stress, and infection by pathogens cause skin inflammation.

Severe inflammation of the skin is typically accompanied by redness, swelling, oozing, scaling and itching.

An estimated 20-25% of the population is affected by inflammatory skin conditions or diseases.

Among the most frequent inflammatory skin diseases are dermatitis, psoriasis, urticaria, lichen planus, pityriasis rosea of Gibert, rosacea and hidradenitis suppurativa.

In dermatitis, or eczema, the stratum corneum, composed of the differentiated keratinocytes called corneocytes, is often compromised. As allergens penetrate the defective skin barrier, pro-inflammatory cytokines, in particular interleukins (IL), are released.

Due to elevated levels of inflammatory cytokines, inflamed skin is at the same time more susceptible to infections, thus fueling a vicious cycle.

Seborrheic dermatitis (SD) is a dermatitis affecting the scalp, face, and body folds. It presents with a papulosquamous morphology in affected areas, that are rich in sebaceous glands. When sebum is produced in greater quantity than normal, it favors the proliferation of fungi of the genus *Malassezia* which feed on lipids and consequently produce large amount of lipase and phospholipase enzymes. The inflammation is then the consequence of the production of molecules capable of triggering oxidative mechanisms that in turn trigger the production of proinflammatory cytokines by keratinocytes and release of histamine by mast cells.

Treatments of skin disorders therefore are typically aimed at restoring the skin barrier and modulating the abnormal immune response.

Currently, steroids, topical immunosuppressants, topical antibiotics, and antihistamines are used as treatments for inflammatory skin diseases. However, drugs for the treatment of skin disorders can themselves cause skin reactions, thus worsening the skin condition instead of ameliorating it. In particular, if used for a long time, treatments for inflammatory skin diseases can cause swelling striae, skin atrophy, capillary dilation, steroid acne, hirsutism, purpura, and other side effects.

The object of the present invention is therefore the provision of an effective treatment for inflammatory skin diseases, which is also well tolerated by the body and which can also be used for long periods, while minimizing side effects.

### SUMMARY OF THE INVENTION

Said object has been achieved by the composition of the present invention, as reported in the claims.

In another aspect, the present invention relates to a pharmaceutical product comprising said composition and suitable pharmaceutically acceptable excipients.

In another aspect, the present invention relates to said pharmaceutical composition, further comprising at least one further dermatological active agent, preferably selected from further anti-inflammatory agents, cortisonic agents, antibiotics, antihistaminic agents, anti-acne agents, anti-dandruff agents, anti-psoriasis agents, antifungal agents, antibacterial agents, anti-seborrheic agents, keratolytic agents, humectants, anti-free radical agents, antioxidants, vitamins, and mixtures thereof.

Furthermore, the present invention relates to the composition, or the pharmaceutical product comprising the same, for use in the treatment of skin diseases, in particular of inflammatory skin diseases.

For the purposes of the present invention, said skin diseases are preferably ichthyosis, skin microbial infection, dermatophytosis, acne, psoriasis, urticaria, lichen planus, pityriasis rosea, hidradenitis suppurativa, seborrheic dermatitis, allergic dermatosis, scleroderma, contact dermatitis, atopic dermatitis, chronic actinic dermatitis, photodermatoses.

### BRIEF DESCRIPTION OF THE FIGURES

The characteristics and the advantages of the present invention will become clear from the following detailed description, the working examples provided for illustrative purposes and the accompanying figure, wherein:

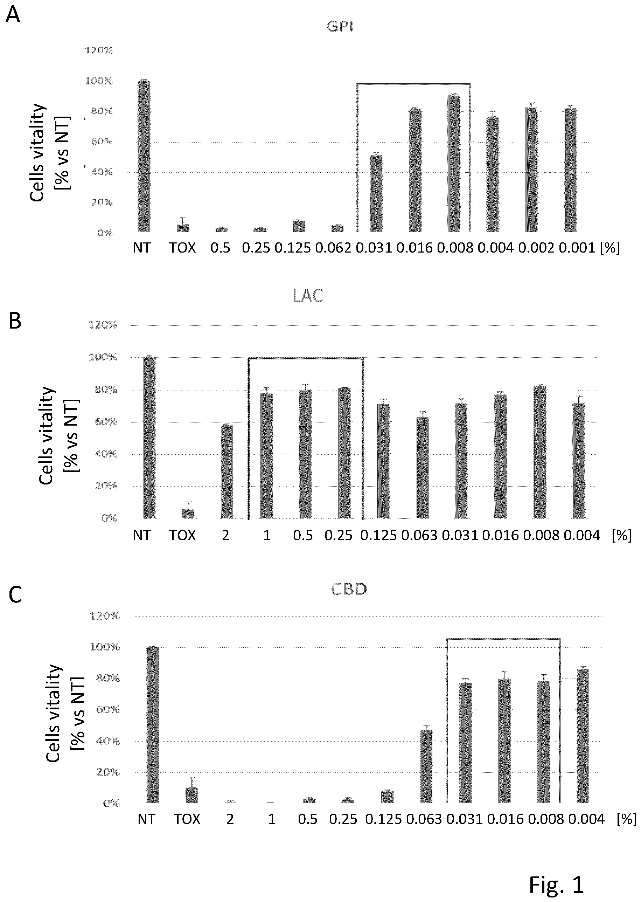

- Fig. 1 shows the results of cytotoxicity tests on HaCaT cells treated for 24 hours at various concentrations of GPI Lysine (A), lactoferrin hydrolysate (B), or cannabidiol (C). Cells vitality is measured as absorbance after MTT treatment. Values are expressed as absorbance percentage over untreated (NT) cells; 1 mg/ml SDS is used as positive control; the boxes show the three highest non-toxic concentrations of each compound.

### DETAILED DESCRIPTION OF THE INVENTION

The composition of the invention comprises:
a) glycerophosphoinositol (GPI), a derivative, or a salt thereof;
b) a lactoferrin hydrolysate; and
c) at least one cannabinoid.

GPI is a natural cell component produced by lipid metabolism.

With reference to ingredient a), preferably, the composition comprises a GPI salt, or a derivative thereof. Preferably, the GPI salt is a choline, arginine, or lysine salt of GPI, or a derivative thereof; more preferably the GPI salt is GPI lysine, or a derivative thereof. A derivative of GPI is preferably glycerophosphoinositol 4-phosphate or glycerol-phosphoinositol 4,5-bisphosphate.

With reference to ingredient b), digestion of lactoferrin can generate bioactive peptides. Lactoferrin hydrolysates then comprise useful bioactive compounds. Thus, the composition of the present invention also comprises a lactoferrin hydrolysates, said hydrolysate comprising lactoferrin peptides obtained by hydrolysation of lactoferrin. Lactoferricin is a cationic peptide which can be generated by digestion mediated by lactoferrin pepsin. Bovine and human lactoferricin have been mostly studied.

The bovine lactoferricin sequence corresponds to the 17-41 fragment of lactoferrin (FKCRRWQWRM KKLGAPSITCVRRAF; LFB0084). In humans, lactoferricin corresponds to lactoferrin fragment 1-47, but consisting of two subunits connected by a disulphide bridge, i.e. fragments 1-11 and 12-47.

Lactoferrampin is a cationic peptide characterised by a highly positive charge and a hydrophobic domain, and therefore an amphipathic character. It comprises residues 268-284 and is located in the N1-domain of lactoferrin, in proximity to lactoferricin. According to preferred embodiments, lactoferrin hydrolysates in the composition of the present invention comprise, or consist of, lactoferricin, or lactoferrampin, or mixtures thereof.

Lactoferricin and lactoferrampin can be obtained by enzymatic hydrolysis of lactoferrin. Suitable enzymes belong to the class of hydrolases which catalyse the breakage of the peptide bond between two consecutive amino acids of the protein in question, in this case lactoferrin. These enzymes exhibit different selectivity towards the different amino acids present, therefore the complete degradation of the protein in the individual amino acid constituents does not take place but rather the generation of peptide fragments of various lengths depending on the position of the amino acids recognised by the hydrolytic enzyme used.

Since the enzyme used for hydrolysis can represent an impurity in the product, preferably lactoferrin hydrolysates are obtained by using enzymes that are immobilised on inert substrate through covalent bonds; immobilisation enables removal of the biocatalyst, at the end of the reaction, by using physical methods (e.g. filtration) and therefore prevents changes in pH and increases in temperature, which are necessary to inactivate the free enzyme but have a negative impact on the activity of the product.

Preferred enzymes used to obtain a lactoferrin hydrolysate are proteases, in particular endoproteases including, preferably, pepsin, clostripain, proteases type XVII, ASP-N endopeptidases, ARG-C proteinases, glutamyl endopeptidases, proteinases, trypsin, thermolysine, subtilisin, chymotrypsin, and mixtures thereof.

In preferred embodiments, the composition of the invention comprises lactoferrin hydrolysate obtained by lactoferrin hydrolisys by means of pepsin, more preferably of pork, or clostripain protease type XVII, ASP-N endoproteases, ARG-C endoproteases, or mixtures thereof.

Preferred lactoferrin hydrolysates, and methods to obtain the same, are those described in WO 2019/021175 A1.

With reference to ingredient c), cannabinoids according to the present invention can be of natural or, preferably, of synthetic origin. Synthetic cannabinoids can be produced semi-synthetically, i.e. from natural cannabinoids, or fully synthetically, i.e. from simple basic substances, such as for example limonene.

Preferably, the at least one cannabinoid is cannabidiol (CBD), cannabidiolic acid (CBDA), cannabinol (CBN), cannabigerol (CBG), cannabichromene (CBC), cannabicyclol (CBL), cannabivarin (CBV), tetrahydrocannabivarin (THCV), cannabidivarin (CBDV), cannabichromevarin (CBCV), cannabigervarin (CBGV), cannabigerol monomethyl ether (CBGM), cannabielsoin (CBE), cannabicitran (CBT), or any mixture thereof.

Preferably, the at least one cannabinoid is cannabigerol (CBG) or cannabidiol (CBD), or a mixture thereof. More preferably, the at least one cannabinoid is cannabidiol (CBD). According to preferred embodiments, the composition of the invention comprises:
a) a glycerophosphoinositol (GPI) salt, or derivative thereof;
b) a lactoferrin hydrolysate comprising lactoferricin, or lactoferrampin, or mixtures thereof; and
c) at least one cannabinoid being CBD.

According to particularly preferred embodiments, the composition of the invention comprises:
a) GPI lysine;
b) a lactoferrin hydrolysate comprising lactoferricin; and
c) CBD.

Preferably, in the composition of the present invention, the GPI, or derivative thereof, or salt thereof, and the lactoferrin hydrolysate are in a weight ratio of from 1:1 to 1:10, more preferably of from 1: 1.5 to 1:5, most preferably of from 1:1.5 to 1:2.

Preferably, in the composition of the present invention, the at least one cannabinoid and the lactoferrin hydrolysate are in a weight ratio of from 1:0.5 to 1:5, more preferably of about 1:0.5 to 1:3, most preferably of from 1:0.5 to 1:1.

Preferably, in the composition of the present invention, the GPI, or derivative thereof, or salt thereof, and the at least one cannabinoid are in a weight ratio of from 1:1 to 1:10, more preferably of from 1:1 to 1:5, most preferably of from 1:2 to 1:3.

According to preferred embodiments, the composition of the present invention comprises a concentration by weight of GPI, or derivative thereof, or salt thereof, of from 0.01 to 1%, more preferably of from 0.05 to 0.5%, most preferably of about 0.1%, based on the total weight of the composition.

According to further, or alternative, embodiments, in the composition of the present invention, the concentration by weight of the lactoferrin hydrolysate is of from 0.1 to 2%, more preferably from 0.1 to 1%, most preferably of from 0.1 to 0.2 %, based on the total weight of the composition.

According to further, or alternative, embodiments, in the composition of the present invention, the concentration by weight of the at least one cannabinoid is of from 0.01 to 0.5%, more preferably from 0.1 to 0.5%, most preferably of about 0.25%, based on the total weight of the composition.

The total weight of the composition is preferably the sum of the weight of ingredients a), b) and c) and the weight of suitable vehicle(s) present in the composition. Suitable vehicle(s) can be for instance water, surfactants, or mixtures thereof.

In accordance with the present invention the terms "wt %" or "% w/w" or "concentration by weight", when referring to the percentage of a component in a composition or product, mean the percentage of the weight of the component, relative to the total weight of the composition or product. The terms "% v/v" or "concentration by volume", when referring to the percentage of a component in a composition or product, mean the percentage of the volume of the component, relative to the total volume of the composition or product. The terms "w/v %", when referring to the percentage of a component in a composition or product, mean the percentage of the weight of the component, relative to the total volume of the composition or product.

Preferably, the composition of the invention is a liquid or semi-liquid solution, comprising:
a) 0.01-0.5 % w/v of glycerophosphoinositol (GPI), or a derivative thereof, or a salt thereof;
b) 0.1-1 % w/v of a lactoferrin hydrolysate; and
c) 0.01-0.5 % w/v of at least one cannabinoid.

According to more preferred embodiments, the composition comprises:
a) 0.05-0.2 % w/v of glycerophosphoinositol (GPI), or a derivative thereof, or a salt thereof;
b) 0.1-0.5 % w/v of a lactoferrin hydrolysate; and
c) 0.1-0.5 % w/v of at least one cannabinoid.

In preferred embodiments, a) glycerophosphoinositol (GPI), a derivative, or a salt thereof, b) a lactoferrin hydrolysate, and c) at least one cannabinoid, are the only active ingredients for the treatment of inflammatory skin conditions or diseases, that are present in the composition of the invention.

The invention further relates to the composition of the invention for use in the treatment of skin diseases, preferably for use as anti-inflammatory agent in the treatment of inflammatory skin diseases.

The term "anti-inflammatory agent" refers to a substance that directly or indirectly reduces inflammation in a tissue.

The term "treatment" refers to the effects of the composition of the invention, which is capable of providing a benefit to patients suffering from a skin disease, for example, an improvement in the patient's condition or a delay in the progression of the disease.

Said skin diseases preferably include ichthyosis, skin microbial infection, dermatophytosis, acne, psoriasis, urticaria, lichen planus, pityriasis rosea, hidradenitis suppurativa, seborrheic dermatitis, allergic dermatosis, scleroderma, contact dermatitis, atopic dermatitis, chronic actinic dermatitis, and photodermatoses.

Preferably, the composition of the invention is for use in the treatment of dermatitis, more preferably of seborrheic dermatitis.

The composition of the invention has proved in fact to be effective in reducing inflammation triggered by an inflammatory insult in keratinocytes, as demonstrated in the examples provided hereby, by reduction of inflammation marker IL-6 after TNF-alpha stimulus.

During chronic inflammation, IL-6 suppression can decrease tissue injury.

The reduction of IL-6 levels achieved with the composition of the invention is greater than that obtained with the same concentrations of the individual ingredients of the composition, demonstrating an unexpected synergic anti-inflammatory effect of the composition of the invention.

In preferred embodiments, a) glycerophosphoinositol (GPI), a derivative, or a salt thereof, b) a lactoferrin hydrolysate, and c) at least one cannabinoid, are the only active ingredients for the treatment of inflammatory skin conditions or diseases, that are present in the composition of the invention.

Preferably, the composition of the invention for use in the treatment of inflammatory skin diseases is to be administered via topical route, more preferably via external topical, subcutaneous topical, mucosal topical, gingival topical, intravesical topical, vaginal topical, rectal topical, or ocular topical route.

Preferably, said composition is to be administered in a dose of 0.1-1500 mg per day, the effective dosage being a function of the extent and severity of the skin disease to be treated.

In preferred embodiments, said composition is to be administered via external topical route in a dose of 1-1000 mg per day.

In further aspects, the present invention relates to a pharmaceutical product comprising the composition of the invention and pharmaceutically acceptable excipients.

The term "excipient" herein means any substance, not itself an active agent, which may be used as a carrier or vehicle for delivery of an active agent to a subject, or combined with an active agent to improve its handling or storage properties, or to permit or facilitate formation of a dose unit of the pharmaceutical product.

Said pharmaceutically acceptable excipients can be rheological additives, buffering agents, antioxidant agents, anti-isothermal agents, antistatic agents, absorbent agents, UV absorbing agents, astringent agents, chelating agents, skin conditioning agents, preservative agents, covering agents, denaturing agents, depigmenting agents, emulsifying agents, film-forming agents, gelling agents, moisturizing agents, hydrotropic agents, binders, soothing agents, smoothing agents, opacifying agents, plasticizing agents, propelling agents, skin protecting agents, reducing agents, cooling agents, sebum-restoring agents, solvents , stabilizing agents, emulsifying stabilizing agents, toning agents, wetting agents, volumizing agents or combinations thereof.

Preferably, the pharmaceutical product of the invention comprises at least one surfactant, more preferably at 1-15% (w/w).

According to preferred embodiments, the pharmaceutical product of the invention, further comprises one or more of: a humectant, preferably at 1 - 10% (w/w); a thickening agent, preferably at 0.01 - 10% (w/w); a gel-forming agent, preferably at 0.1 -5% (w/w); a preservative, preferably at 0.1 -3% (w/w); an emollient, preferably at 1-3% (w/w); a penetration enhancer, preferably at 1-5% (w/w); a pH adjusting agent, preferably in a quantity sufficient for the composition to maintain a pH of 5-7; and water to make up 100% by weight.

The pharmaceutical product of the invention preferably comprises the active ingredients, lactoferrin hydrolysate, GPI, or derivative thereof, or salt thereof, and at least one cannabinoid, in the ratios and/or concentrations described above with reference to the composition of the invention.

Therefore, preferably the pharmaceutical product of the invention comprises:
- the GPI, or derivative thereof, or salt thereof, in a weight ratio with the lactoferrin hydrolysate of from 1:1 to 1:10, more preferably of from 1:1.5 to 1:5, most preferably of from 1:1.5 to 1:2; and/or
- the at least one cannabinoid in a weight ratio with the lactoferrin hydrolysate of from 1:0.5 to 1:5, more preferably of about 1:0.5 to 1:3, most preferably of from 1:0.5 to 1:1; and/or
- the GPI, or derivative thereof, or salt thereof, in a weight ratio with the at least one cannabinoid of from 1:1 to 1:10, more preferably of from 1:1 to 1:5, most preferably of from 1:2 to 1:3.

According to preferred embodiments, the pharmaceutical product of the present invention comprises a concentration by weight of GPI, or derivative thereof, or salt thereof, of from 0.01 to 1%, more preferably of from 0.05 to 0.5%, most preferably of about 0.1%, based on the total weight of the pharmaceutical product.

According to further, or alternative, embodiments, in the pharmaceutical product of the present invention, the concentration by weight of the lactoferrin hydrolysate is of from 0.1 to 2%, more preferably from 0.1 to 1%, most preferably of from 0.1 to 0.2 %, based on the total weight of the pharmaceutical product.

According to further, or alternative, embodiments, in the pharmaceutical product of the present invention, the concentration by weight of the at least one cannabinoid is of from 0.01 to 0.5%, more preferably from 0.1 to 0.5%, most preferably of about 0.25%, based on the total weight of the pharmaceutical product.

The total weight of the pharmaceutical product is preferably the sum of the weight of ingredients a), b) and c) and the weight of the pharmaceutically acceptable excipients present in the product.

Preferably, the pharmaceutical product of the invention comprises:
d) 0.01-0.5 % w/v of glycerophosphoinositol (GPI), or a derivative thereof, or a salt thereof;
e) 0.1-1 % w/v of a lactoferrin hydrolysate; and
f) 0.01-0.5 % w/v of at least one cannabinoid.

According to further preferred embodiments, the pharmaceutical product of the invention comprises:
- a concentration by weight of GPI, or a derivative thereof, or a salt thereof, of from 0.05 to 0.2%; and/or
- a concentration by weight of the lactoferrin hydrolysate of from 0.1 to 0.5%; and/or
- a concentration by weight of the at least one cannabinoid of from 0.1 to 0.5%,

based on the total weight of the composition,
and pharmaceutically acceptable excipients.

Preferably, in the pharmaceutical product of the invention, a) GPI, or a derivative thereof, or a salt thereof, is a GPI salt, more preferably GPI lysine. Optionally, the pharmaceutical product of the invention comprises an aqueous mixture of GPI lysine and ethylhexylglycerin, said mixture comprising about 10% by weight of GPI lysine and about 0.1% of ethylhexylglycerin. Such a mixture is commercially available under the name of "GPI lysine PE". More preferably, the pharmaceutical product of the invention comprises a concentration by volume of GPI lysine PE of 0.5-2%, more preferably of about 1%, based on the total volume of the pharmaceutical product.

According to further, or alternative, embodiments, the composition of the present invention comprises a concentration by volume of the lactoferrin hydrolysate is of from 0.1 to 2%, more preferably from 0.1 to 1%, most preferably of about 0.5%, based on the total volume of the composition.

According to further, or alternative, embodiments, the composition of the present invention comprises a concentration by volume of the at least one cannabinoid is of from 0.01 to 0.5%, more preferably from 0.1 to 0.5%, most preferably of about 0.25%, based on the total volume of the composition.

Preferably, in the pharmaceutical product of the invention, the lactoferrin hydrolysate is a hydrolysate comprising, or consisting of, lactoferricin, lactoferrampin, or a mixture thereof. More preferably, in the pharmaceutical product of the invention, the lactoferrin hydrolysate is a hydrolysate comprising, or consisting of, lactoferricin.

Preferably, in the pharmaceutical product of the invention, c) the at least one cannabinoid is cannabidiol.

Preferred pharmaceutical products according to the present invention are shown in tables 1-4 that follow, wherein tables 1-3 refer to products in the form of mousse, while table 4 refers to a product in the form of a serum:

**Table 1**

| **Dermatological mousse** | |
|---|---|
| **Ingredients** | **w/v %** |
| Polysorbate 20 | 2 |
| Polyglyceryl-3 Cocoate | 1.9 |
| Sodium Lauroyl Sarcosinate | 1.5 |
| Acrylates/C10-30 Alkyl Acrylate Crosspolymer | 0.25 |
| Cannabidiol | 0.25 |
| Lactoferrin hydrolysate | 0.2 |
| GPI Lysine | 0.1 |
| Water | balance to 100 |

**Table 2**

| **dermatological mousse** | |
|---|---|
| **Ingredients** | **w/v %** |
| Sodium Lauroyl Sarcosinate | 2 |
| Polysorbate 20 | 2 |
| Polyglyceryl-3 Cocoate | 1.5 |
| Acrylates/C10-30 Alkyl Acrylate Crosspolymer | 0.4 |
| Lactoferrin hydrolysate | 0.3 |
| Cannabidiol | 0.3 |
| GPI Lysine | 0.1 |
| Water | balance to 100 |

**Table 3**

| **dermatological mousse** | |
|---|---|
| **Ingredients** | **w/v %** |
| Polysorbate 20 | 2 |
| Sodium Lauroyl Sarcosinate | 1,5 |
| Polyglyceryl-3 Cocoate | 1.5 |
| Acrylates/C10-30 Alkyl Acrylate Crosspolymer | 0.3 |
| GPI Lysine | 0.2 |
| Lactoferrin hydrolysate | 0.2 |
| Cannabidiol | 0.15 |
| Water | balance to 100 |

Optionally, the pharmaceutical products in form of mousse, further comprise one or more of glycine, phenoxyethanol, ethylhexylglycerin, triethanolamine, contributing with water to balance to 100%.

**Table 4**

| **dermatological serum** | |
|---|---|
| **Ingredients** | **w/v %** |
| Propanediol | 5 |
| Ethanol | 1 |
| Polyglyceryl-3-Laurate | 0.5 |
| Ethylexylglycerin | 0.2 |
| Cannabidiol | 0.2 |
| Lactoferrin hydrolysate | 0.1 |
| GPI Lysine | 0.05 |
| Sodium Benzoate | 0.05 |
| Citric acid | 0.05 |
| Water | balance to 100 |

Preferably, the pharmaceutical product of the invention is prepared by a method comprising the steps of:
- dissolving GPI and the lactoferrin hydrolysate in a buffer solution, preferably in a phosphate buffer solution (PBS);
- dissolving the at least one cannabinoid in a mixture comprising at least one surfactant, preferably comprising polysorbate;
- optionally adding one or more further pharmaceutical excipients to the mixture comprising the at least one cannabinoid;
- mixing under stirring the solution comprising GPI and the lactoferrin hydrolysate with the mixture comprising the at least one cannabinoid and the at least one surfactant, and optionally comprising further pharmaceutical excipients, obtaining the product of the invention.

Preferably, GPI lysine is added to the buffer solution in the form of an aqueous mixture comprising 10% GPI lysine and 0.1% ethylhexylglycerin, that is commercially available under the name of "GPI lysine PE".

Preferably, the pharmaceutical product is to be administered via external topical, subcutaneous topical, mucosal topical, gingival topical, intravesical topical, vaginal topical, rectal topical, or ocular topical route.

In preferred embodiments, said composition is to be administered via external topical route.

Preferably, the pharmaceutical product comprises the composition of the invention in a concentration of 0.1-500 mg/ml of composition, more preferably 1-100 mg/ml.

Said pharmaceutical product may be in the form of ointment, lotion, cream, emulsion, paste, gel, aqueous solution, spray, patch, serum, soaked gauze, dressing, or a combination thereof.

Preferably, the pharmaceutical product further comprises at least one further dermatological active agent, preferably selected from further anti-inflammatory agents, cortisonic agents, antibiotics, antihistaminic agents, anti-acne agents, anti-dandruff agents, anti-psoriasis agents, antifungal agents, antibacterial agents, anti-seborrheic agents, keratolytic agents, humectants, anti-free radical agents, antioxidants, vitamins, and mixtures thereof.

Examples of antifungal agents are econazole and miconazole.

Examples of antibacterial agents are chlorquinol, hexchlorophene, 2,4,4'-trichloro-2'-hydroxybiphenyl ether and usnic acid.

An example of an anti-dandruff agent is ketoconazole.

Examples of anti-isothermal agents are some fatty substances and thiossolone.

Examples of anti-acne agents are retinoic acid and its derivatives.

An example of a keratolytic agent is salicylic acid.

An example of an anti-psoriasis agent is antraline.

All the pharmaceutical compositions described above can be prepared by methods known in the pharmaceutical technique.

In some embodiments, the pharmaceutical product of the invention is for use in the treatment of skin diseases.

The present invention is then directed also to the pharmaceutical product of the invention for use in the treatment of a skin disease.

Said skin diseases preferably: ichthyosis, skin microbial infection, dermatophytosis, acne, psoriasis, urticaria, lichen planus, pityriasis rosea, hidradenitis suppurativa, seborrheic dermatitis, allergic dermatosis, scleroderma, contact dermatitis, atopic dermatitis, chronic actinic dermatitis, photodermatoses.

Preferably, said skin disease is dermatitis, more preferably seborrheic dermatitis.

It should be understood that all the aspects identified as preferred and advantageous for the composition of the invention are to be deemed as similarly preferred and advantageous also for the pharmaceutical product and uses thereof.

Below are working examples of the present invention, provided for illustrative purposes.

### EXAMPLES

### Example 1

Compounds to be tested were prepared by dissolving:
cannabidiol in a mixture of polysorbate and water;
GPI Lysine in PBS;
lactoferrin hydrolysate in PBS.

A mixture of cannabidiol, GPI lysine and lactoferrin hydrolysate was prepared by mixing the above.

Different concentrations of GPI lysine, lactoferrin hydrolysate and cannabidiol, as single ingredients or in admixture, were prepared by serial dilutions.

### Example 2

Cell lines of immortalized human keratinocyes (HaCaT cells) were grown at 37 °C, 5% CO₂ in DMEM culture medium, added with 1% non-essential aminoacids and 10% FBS. The cells were plated in 96-wells multi-well plates at 10⁴ cells/well, in 10% FBS -DMEM. The day after, the culture medium was removed and substituted with 100 µL of complete medium with 1% FBS; then, an MTT assay was carried out after treating cells with the single ingredients prepared in Example 1.

The following concentrations of each ingredient were tested:
- Cannabidiol (CBD): 2, 1, 0.5, 0.25 0.125, 0.063, 0.031, 0.016, 0.008, 0.004 % (weight/volume, w/v);
- GPI lysine (GPI): 0.5, 0.25, 0.125, 0.0625, 0.0313, 0.0156, 0.0078, 0.0039, 0.002, 0.001 % (w/v);
- lactoferrin hydrolysate (LAC): 2, 1, 0.5, 0.25 0.125, 0.063, 0.031, 0.016, 0.008, 0.004 % (w/v).

As positive control of toxicity, sodium dodecyl sulfate (SDS) was used, at the concentration of 1 mg/ml.

The cells were incubated for 24 hours at 37°C and 5% CO₂.

At the end of each incubation, the reagent 3-(4,5-dimethylthiazol-2-yl)-2,5-diphenyltetrazolium bromide (MTT) was added to each well, at the final concentration of 0.45 mg/mL. The plates were then incubated for an additional 2 hours, at 37 °C and 5% CO₂.

At the end of the incubation, the crystals of reduced MTT were solubilized by removing the medium and adding 100 µL of dimethyl sulfoxide (DMSO) to each well.

Finally, the absorbance at 595 nm of each sample was measured, using an Infinite M NANO+ (Tecan) plate reader.

The cell viability following treatment with the different substances was analyzed. Cell viability is expressed as percentage of absorbance at 595 nm of untreated cells (NT). The test results are shown in Fig. 1.

Above 50% viability was observed with GPI Lysine at the concentration of 0.0313 % v/v, while cell viability was stabilized at around 80% at concentrations of 0.0156 - 0.001 % v/v (see Fig. 1A).

LAC showed a good toxicological profile, with cell viability between 60 and 80 % at all the concentrations tested (see Fig. 1B).

A viability of about 50% was observed at a concentration of CBD of 0.063 % w/v, while cell viability stabilized at around 80% at tested concentrations from 0.031 to 0.004 % w/v, with a slight dose-response trend (see Fig. 1C).

### Example 3

Cell lines of immortalized human keratinocytes (HaCaT cells) were grown at 37 °C, 5% CO₂ in DMEM culture medium, added with 1% non-essential aminoacids and 10% FBS. The cells were plated in 48-wells multi-well plates at 5*10⁴ cells/ml, in 10% FBS DMEM. The day after, the culture medium was removed and substituted with 500 µL of complete medium without FBS and at the same time an inflammatory process was induced by treatment with TNFα 20 ng/ml, maintained over/night. This stimulus induces an increase in production of these cytokines compared to controls not receiving TNFα.

An experimental group of untreated cells was included, as a negative control of inflammation.

The following day, the inflamed cells were treated with each ingredient prepared in Example 1, at the highest non-toxic concentrations identified in Example 2.
- Cannabidiol (CBD): 0.031 (w/v)
- GPI lysine (GPI): 0.31 (w/v)
- lactoferrin hydrolysate (LAC): 1 (w/v).

Furthermore, treatment with a mixture of the three ingredients at the same concentrations was carried out, in order to verify effectiveness and synergy of the composition of the invention.

In particular, the following mixture was tested:

**Table 5**

| | CBD [w/v] | GPI [v/v] | LAC [w/v] |
|---|---|---|---|
| Mix | 0.03 | 0.031 | 1 |

After 24 hours of treatment, the culture medium for each well was taken and stored at - 20°C, for subsequent ELISA analyses.

The dosage of the pro-inflammatory cytokine IL-6 (interleukin 6) was performed on 100 µL of each of the stored aliquots, following the kit protocol provided by the manufacturer. IL-6 is a mediator of inflammation that is produced and released by tissues that suffer an insult, with consequent damage to be repaired. Moreover, it is known that patients affected by dermatitis show an altered profile of cytokine expression, with excessive secretion of cytokines such as IL-6, leading to immunological dysregulation. Consequently, the dosage of this cytokine is a valid indicator of inflammatory processes various nature and its reduction is an indicator of effectiveness of potential treatments. The anti-inflammatory effect of the compounds and compositions testes is expressed as a percentage of interleukin's production, with respect to inflamed cells (TNFα-treated) not subjected to anti-inflammatory treatment.

The results are shown in Table 6 and 7 that follow.

**Table 6**

| IL-6 production (% vs TNFα) | NT | TNFα | LAC 1% (w/v) | CBD 0.03% (w/v) | GPI 0.031% (v/v) |
|---|---|---|---|---|---|
| Mean | 4 | 100 | 143 | 99 | 96 |
| St.dev. % | 0.06 | 0.08 | 0.29 | 0.12 | 0.18 |

**Table 7**

| IL-6 production (% vs TNFα) | NT | TNFα | **MIX** |
|---|---|---|---|
| Mean | 4 | 100 | **48** |
| St.dev. % | 0.06 | 0.08 | **0.04** |

None of the single ingredients' treatment led to a reduction of cell's production of IL-6. However, when the ingredients were mixed together, the inflammatory process was reduced, thus demonstrating a synergic anti-inflammatory activity of compositions according to the present invention.

Reduction of inflammation in keratinocytes subject to inflammatory insult, by the composition of the invention, is predictive of efficacy of the composition in treating inflammatory skin diseases.

### Example 4

Pharmaceutical products according to the invention for *in vivo* topical administration was prepared, by adapting the concentrations of the ingredients of the synergic compositions tested *in vitro* in Examples 2 and 3.

In particular, cannabidiol, previously dissolved in a mixture of polysorbate 20 and water, was mixed together with GPI Lysine and lactoferrin hydrolysate, previously dissolved in PBS, and with further pharmaceutical acceptable excipients, to obtain a final concentration of: 0.2-0.5 % w/v cannabidiol, 0.05-0.2% w/v GPI lysine, 0.05-0.5% lactoferrin hydrolysate, based on the total volume of the pharmaceutical product.

## Claims

1. An anti-inflammatory composition comprising:
a) glycerophosphoinositol (GPI), or a derivative thereof, or a salt thereof;
b) a lactoferrin hydrolysate; and
c) at least one cannabinoid.

2. The anti-inflammatory composition of claim 1, wherein a) the glycerophosphoinositol (GPI), or a derivative thereof, or a salt thereof, is a GPI salt, preferably GPI lysine, or a derivative thereof.

3. The anti-inflammatory composition of any one of claims 1-2, wherein b) the lactoferrin hydrolysates comprises, or consist of, lactoferricin, or lactoferrampin, or mixtures thereof.

4. The anti-inflammatory composition of any one of claims 1-3, wherein c) the at least one cannabinoid is selected from: cannabidiol (CBD), cannabidiolic acid (CBDA), cannabinol (CBN), cannabigerol (CBG), cannabichromene (CBC), cannabicyclol (CBL), cannabivarin (CBV), tetrahydrocannabivarin (THCV), cannabidivarin (CBDV), cannabichromevarin (CBCV), cannabigervarin (CBGV), cannabigerol monomethyl ether (CBGM), cannabielsoin (CBE), cannabicitran (CBT), and combinations thereof; preferably wherein c) the at least one cannabinoid is cannabigerol (CBG) or cannabidiol (CBD), or mixtures thereof; more preferably wherein c) the at least one cannabinoid is cannabidiol (CBD).

5. The anti-inflammatory composition of any one of claims 1-4, wherein:
- the GPI and the lactoferrin hydrolysate are in a weight ratio of from rom 1:1 to 1:10, preferably of from 1:1.5 to 1:5, more preferably of from 1:1.5 to 1:2.; and/or
- the at least one cannabinoid and the lactoferrin hydrolysate are in a weight ratio of from 1:0.5 to 1:5, preferably of about 1:0.5 to 1:3, more preferably of from 1:0.5 to 1:1; and/or
- the GPI and the at least one cannabinoid are in a weight ratio of from 1:1 to 1:10, preferably of from 1:1 to 1:5, more preferably of from 1:2 to 1:3.

6. The anti-inflammatory composition of any one of claims 1-5 comprising:
- a concentration by weight of GPI, or a derivative thereof, or a salt thereof, of from 0.01 to 1% w/v, preferably from 0.05 to 0.5%, more preferably of about 0.1%, based on the total weight of the composition; and/or
- a concentration by weight of the lactoferrin hydrolysate of from 0.1 to 2%, preferably from 0.1 to1%, more preferably of 0.1 to 0.2%, based on the total weight of the composition; and/or
- a concentration by weight of the at least one cannabinoid of from 0.01 to 0.5%, preferably from 0.1 to 0.5%, more preferably of 0.25%, based on the total weight of the composition.

7. A pharmaceutical product comprising the composition of any one of claims 1-6, and pharmaceutically acceptable excipients.

8. The pharmaceutical product of claim 7, comprising:
- a concentration by weight of GPI, or a derivative thereof, or a salt thereof, of from 0.05 to 0.2%; and/or
- a concentration by weight of the lactoferrin hydrolysate of from 0.1 to 0.5%; and/or
- a concentration by weight of the at least one cannabinoid of from 0.1 to 0.5%,
based on the total weight of the composition,
and pharmaceutically acceptable excipients.

9. The pharmaceutical product of any one of claims 7-8, further comprising at least one further dermatological active agent, preferably selected from: cortisonic agents, antibiotics, antihistaminic agents, anti-acne agents, anti-dandruff agents, anti-psoriasis agents, antifungal agents, antibacterial agents, anti-seborrheic agents, keratolytic agents, humectants, anti-free radical agents, antioxidants, vitamins, and mixtures thereof.

10. The anti-inflammatory composition of any one of claims 1-6 or the pharmaceutical product of any one of claims 7-9, for use in the treatment of a skin disease.

11. The anti-inflammatory composition of any one of claims 1-6 or the pharmaceutical product of any one of claims 7-9, for use in the treatment of a skin disease selected from the group consisting of: ichthyosis, skin microbial infection, dermatophytosis, acne, psoriasis, urticaria, lichen planus, pityriasis rosea, hidradenitis suppurativa, seborrheic dermatitis, allergic dermatosis, scleroderma, contact dermatitis, atopic dermatitis, chronic actinic dermatitis, and photodermatoses.
